(19) Europäisches Patentamt
European Patent Office
Office européen des brevets

(11) **EP 0 750 009 B1**

(12) **FASCICULE DE BREVET EUROPEEN**

(45) Date de publication et mention
de la délivrance du brevet:
**01.12.2004  Bulletin 2004/49**

(51) Int Cl.$^7$: **C08K 5/07**, C08L 57/08

(21) Numéro de dépôt: 96401217.3

(22) Date de dépôt: 06.06.1996

(54) **Composition pour polymère chloré à base de béta-dicétone et d'acétylacétonate**

Zusammensetzung für Chlorpolymer auf Basis von Beta-Diketon und Acetylacetonat

Composition for chlorinated polymer based on betadiketone and acetylacetonate

(84) Etats contractants désignés:
**AT BE CH DE DK ES FI FR GB GR IE IT LI LU NL PT SE**

(30) Priorité: **14.06.1995  FR 9507089**

(43) Date de publication de la demande:
**27.12.1996  Bulletin 1996/52**

(73) Titulaire: **RHODIA CHIMIE
92408 Courbevoie Cédex (FR)**

(72) Inventeurs:
• **Henrio, Françoise
78630 Morainvilliers (FR)**
• **Gay, Michel
69100 Villeurbanne (FR)**

• **Mur, Gilles
94100 Saint Maur des Fosses (FR)**

(74) Mandataire: **Bernasconi, Jean et al
c/o Cabinet Lavoix,
2, Place d'Estienne d'Orves
75441 Paris Cédex (FR)**

(56) Documents cités:
**EP-A- 0 596 809        EP-A- 0 658 592
DE-A- 4 134 325**

Remarques:
Le dossier contient des informations techniques présentées postérieurement au dépôt de la demande et ne figurant pas dans le présent fascicule.

**Description**

La présente invention concerne des compositions pour polymère chloré comprenant une composition stabilisante ainsi que les objets conformes obtenus à partir desdites compositions.

Les béta-dicétones font partie aujourd'hui des meilleurs stabilisants organiques pour les polymères chlorés tels que le PVC. C'est pourquoi ces composés sont devenus de plus en plus importants sur le plan commercial.

Aussi le coût de fabrication de ces béta-dicétones est un facteur important qui peut en particulier limiter leur développement. En effet, il est tout à fait économiquement impensable d'utiliser des stabilisants trop coûteux dans l'industrie des polymères chlorés et notamment celle du PVC.

Par ailleurs, pour stabiliser ce type de polymère, on connait également l'utilisation de complexes métalliques de composés dicarbonylés. Il s'agit en particulier de chélates de calcium ou de zinc de composés susceptibles de tautomérisme céto-énolique tel que des béta-cétoesters ou des béta-dicétones.

Parmi ces composés, on peut citer l'acétylacétate d'éthyle ou l'acétylacétone.

Ces chélates entrent en général dans des compositions plus complexes qui comprennent aussi d'autres constituants tels que des sels de calcium ou de zinc éventuellement associés à un autre accepteur de HCl, des composés surbasiques (phénolate, sulfonate), hydrotalcites, phosphites, orthoesters, polyols, etc.

Plus récemment, il a été décrit dans la demande de brevet DE-A-4 134 325 (HENKEL) des compositions stabilisantes comprenant notamment un complexe de calcium d'une béta-dicétone tel que l'acétylacétonate de calcium, un sel de zinc, une béta-dicétone, un co-stabilisant minéral et un polyol.

L'objectif de la présente invention est de fournir une composition présentant des propriétés améliorées quant à son action stabilisante du point de vue thermique et des rayons ultra-violets, qui soit aisément disponible et peu coûteuse.

A cette fin, l'invention a pour objet une composition pour polymère chloré tel que notamment le PVC, caractérisée en ce qu'elle comprend, en tant que composition stabilisante :

a) le produit brut non purifié résultant de la réaction de condensation d'un ester sur une cétone en présence d'un agent alcalin, ce produit brut comportant entre 10 et 95 %, de préférence entre 20 et 80 % en poids de béta-dicétone, et

b) un complexe métallique d'acétylacétonate,

le rapport des constituants a/b étant compris entre 50/50 et 1/99.

L'invention est également relative aux objets conformes obtenus à partir de cette composition.

Après avoir effectué de longues et coûteuses recherches, la Demanderesse a trouvé de façon totalement inattendue qu'une composition stabilisante pour polymère chloré (PVC) comprenant le produit brut non purifié (a) résultant de la réaction de condensation d'un ester sur une cétone en présence d'un agent alcalin, en association avec de l'acétylacétonate, présente, à poids identique, une action stabilisante au moins égale à celle qu'aurait eu une composition stabilisante similaire comportant de la béta-dicétone purifiée recristallisée, toute chose étant égale par ailleurs.

Le produit brut précité comprend entre 10 et 95 %, de préférence entre 20 et 80 % en poids de béta-dicétone et peut être utilisé sous forme solide.

La réaction de condensation d'un ester sur une cétone peut s'écrire :

$$R_1COCHR_2H + R_3CO\text{-}OR_4 + ACat \rightarrow R_1COCHR_2COR_3 + R_4OH$$

avec :

- A Cat est choisi parmi un amidure d'un cation ou un hydrure d'un cation.
- $R_1$ et $R_3$, qui peuvent être semblables ou différents, représentent chacun un radical hydrocarboné, ayant avantageusement de 1 à 30 atomes de carbone, de préférence de 1 à 18 atomes de carbone ;
- $R_2$ est un hydrogène ou un radical hydrocarboné, en général alkyle, présentant avantageusement au plus 4 atomes de carbones ;
- $R_1$ et $R_2$ peuvent être reliés de manière que la béta-dicétone forme un cycle ;
- $R_4$ représente un radical hydrocarboné.

En ce qui concerne $R_1$, $R_2$ et $R_3$, on peut utiliser un large spectre de radicaux.

Aussi $R_1$ et $R_3$, qui sont identiques ou différents, peuvent représenter :

- un radical aralkyle ou alcényle, linéaire ou ramifié ayant jusqu'à 24 atomes de carbone ;

- un radical aralkyle ayant de 7 à 10 atomes de carbone ;
- un radical aryle ou cycloaliphatique ayant moins de 14 atomes de carbone, les radicaux cycloaliphatiques pouvant éventuellement comporter des liaisons doubles carbone-carbone.

Ces radicaux peuvent être substitués ou non, par exemple par des atomes d'halogène ou, pour des radicaux aryles ou cycloaliphatiques, par des groupes méthyle ou éthyle.

Les radicaux précédemment énumérés peuvent aussi être modifiés par la présence dans la chaîne aliphatique d'un ou plusieurs des groupements de formule :

$$- O-, -CO-O-, -CO-$$

$R_1^-$ et $R_3$ peuvent également représenter, ensemble, un radical unique divalent ayant de 2 à 5 atomes de carbone et pouvant comporter un hétéroatome d'oxygène ou d'azote ;

$R_2$ peut être :

- un atome d'hydrogène (cas préféré) ;
- un radical alkyle, substitué ou non, ayant de 1 à 4 atomes de carbone et pouvant être interrompu par des groupes -O-, -CO-O, -CO-.

$R_4$ représente un radical alkyle ayant de 1 à 4 atomes de carbone, de préférence méthyle.

La cation Cat est généralement un métal alcalin, de préférence le sodium.

La réaction de condensation décrite ci-dessus est bien connue de l'homme du métier et est plus particulièrement décrite, notamment dans les publications suivantes :

- R. HAUSER et Coll., "The acylation of ketones to form diketones", ORGANIC REACTIONS - Vol. VII, Chapter 3, p.59 - 196, John WILER, Ed. NEW-YORK (1954)
- WIEDMAN et Coll., C.R. 238 (1954), p. 699
- MORGAN Et Coll., Ber. 58 (1925), p. 333
- LIVINGSTONE et Coll., Am. Soc. 46 (1924), p. 881-888
- LEVINE Robert et Coll., Am. Soc. 67 (1945), p. 1510-1517

qui sont incorporés comme références dans la présente description.

Selon un mode de réalisation préféré, le choix des réactifs de départ est basé sur la disponibilité de ces produits et sur l'activité des produits réactionnels obtenues ; on utilise ainsi un ester comme produit de départ préféré :

- pour l'ester, du stéarate de méthyle notamment de qualité technique pouvant alors contenir d'autres esters d'acides gras dont en particulier, le palmitate de méthyle,
- pour l'acétone, l'acétophénone et,
- pour l'agent alcalin, l'amidure de sodium.

On recommande d'utiliser 2 moles d'amidure par mole d'ester ou de cétone engagé et d'utiliser un léger excès (entre 5 et 30 %) molaire de cétone par rapport à l'ester.

Du fait de la présence d'amidure de sodium, il est préférable d'effectuer la réaction sous atmosphère inerte, de préférence sous balayage d'azote.

La réaction s'effectue à une température située de préférence entre 30 et 60°C. A la température ambiante (20°C), la cinétique est trop lente. En outre si la température est trop élevée, par exemple à 60°C et plus, une telle température favorise d'une part, l'autocondensation des cétones en général et de l'acétophénone en particulier et, d'autre part, la formation d'amides.

Les solvants utilisables sont les solvants inertes du genre éther, notamment l'éther isopropylique, les hydrocarbures aliphatiques (par exemple le cyclohexane) ou bien les hydrocarbures aromatiques (toluène).

Bien qu'il soit techniquement possible de conduire la réaction sous une pression plus élevée que la pression atmosphérique, on préfère, pour des motifs économiques, travailler à pression atmosphérique ou bien sous pression réduite de manière à abaisser les températures indiquées ci-dessus et les amener à un domaine compris entre 35 et 55°C. On utilise rarement des pressions inférieures à $10^4$ Pa.

En fin de réaction, le milieu est acidifié. Pour ce faire, on coule la solution réactionnelle dans une solution aqueuse d'un acide dont les préférés sont l'acide acétique, l'acide chlorhydrique et l'acide sulfurique. On ajuste le pH de la couche aqueuse à une valeur comprise de préférence entre 1 et 3.

Pour l'introduction des réactifs, on peut en particulier envisager les 3 modes opératoires suivants :

a) on forme au préalable, l'anion énolique de l'acétophénone en coulant l'acétophénone dans le mélange amidure/ solvant, puis on additionne l'ester,

b) on charge le solvant, l'amidure et la totalité de l'ester, puis on coule lentement l'acétophénone,

c) on coule simultanément l'acétophénone et l'ester dans le mélange amidure/solvant.

On recommande d'utiliser la voie b) puis d'acidifier le mélange réactionnel dans un excès molaire (1,2 à 2 fois molaire) d'acide sulfurique dilué à 5-20 % dans l'eau de façon à ce que le pH soit d'environ 1,5.

Après au moins un lavage à l'eau, on élimine le solvant par tout moyen convenable, par exemple par évaporation et on obtient un produit brut solide à température ambiante comportant généralememnt entre 40 % et 90 % en poids de béta-dicétone.

Selon une variante de l'invention, ce produit brut, une fois broyé et réduit à l'état de poudre, est directement utilisable comme additif dans une composition stabilisante pour polymère chloré.

Cette poudre présente une granulométrie généralememnt inférieure à 500 µm, de préférence inférieure à 200 µm.

De façon à aboutir à la granulométrie souhaitée, on peut utiliser toutes les techniques connues de l'homme du métier pour obtenir des poudres et notamment :

a) la précipitation dans un solvant,

b) le broyage cryogénique,

c) la pulvérisation/séchage dans un courant froid à contre courant ou à cocourant.

Selon la technique a), on dissout le produit brut solide de la réaction dans un solvant approprié comme par exemple l'éthanol ou le méthanol à température ambiante, on distille le solvant sous une pression réduite de l'ordre de $10^3$ Pa puis on injecte de l'azote.

Selon la technique b), on introduit dans un broyeur de l'azote liquide et le produit brut réactionnel sous forme de morceaux de quelques millimètres à quelques centimètres obtenus par broyage grossier ou bien par la technique "d'écaillage" de la solution réactionnelle. "L'écaillage" permet d'éliminer le solvant de cette solution réactionnelle par passage de la solution sur un tambour tournant refroidi en permanence. Le produit solidifié à la surface du tambour est récupéré par un racle sous forme précisément "d'écailles". A la place d'azote liquide, on peut utiliser d'autre gaz liquide inerte tel que le $CO_2$ liquide.

Selon la technique c), on pulvérise le produit brut réactionnel à l'état fondu à travers un contrecourant ou un cocourant d'un gaz inerte vis-à-vis du produit tel que de l'air appauvri en oxygène. On récupère des microbilles de produit dont la granulométrie peut être sans difficulté inférieure à 100 µm et aller jusqu'à 10 µm.

Selon une technique bien connue, le produit réactionnel brut peut être recristallisé dans un solvant organique approprié, généralement l'éthanol. Le produit recristallisé, séparé par simple filtration des, jus mères, se présente sous forme de poudre et est essentiellement formé de béta-dicétones. Pour certaines applications, il est nécessaire d'utiliser des béta-dicétones purifiées. L'utilisation des béta-dicétones purifiées à plus de 95 % en poids dans les compositions stabilisantes pour polymère greffé, ne fait pas partie de la présente invention.

Par contre, selon un autre aspect de l'invention, on a trouvé que les jus mères, après élimination du solvant de cristallisation par toute méthode adaptée (par exemple par évaporation ou par la technique d'écaillage citée ci-dessus), conduisent à un résidu solide de recristallisation comportant alors généralement au moins 10 % et le plus souvent entre 20 et 40 % de béta-dicétones et que ce résidu de cristallisation, pouvant être mis sous forme de poudre, présente en combinaison avec de l'acétylacétonate, une action stabilisante sur les polymères similaire à celle qu'aurait eu le même poids d'une composition stabilisante à base de béta-dicétone purifiée recristallisée.

Ces résidus lourds solides de recristallisation sont utilisés de la même façon que le produit brut réactionnel et peuvent être mis en poudre par les mêmes méthodes.

Selon l'invention, le constituant (a) à savoir soit la béta-dicétone non purifiée, soit les résidus lourds de recristallisation, tels que précité, sont utilisés en mélange avec de l'acétylacétonate (b).

L'acétylacétonate est avantageusement sous forme de complexe de calcium ou de zinc.

Il peut s'agir en particulier d'un complexe formé d'une mole d'hydroxyde de calcium $Ca(OH)_2$ et de deux moles d'acétylacétone.

La composition stabilisante conforme à l'invention comprend avantageusement plus d'acétylacétonate (b) que de béta-dicétone non purifiée (a). Le rapport des constituants a/b est compris entre 50/50 et 1/99, et de préférence entre 20/80 et 1/99.

Une stabilisation satisfaisante des polymères chlorés et plus particulièrement du PVC nécessite le plus souvent l'utilisation conjointe de plusieurs stabilisants qui agissent de manière complémentaire et parfois synergétique.

Ainsi, les compositions selon l'invention contiennent de préférence un pigment blanc pour ses qualités optiques. Selon une variante préférée de l'invention, l'addition de TiO$_2$ permet d'améliorer de façon surprenante et substantielle le compromis blancheur/tenue à la lumière et en particulier le phénomène bien connu de rosissement. Conformément à l'invention, on recommande d'utiliser du TiO$_2$ rutile dont la granulométrie est généralement comprise entre 0,1 et 0,5 μm. On prévoit avantageusement l'addition de 0,5 à 10 parties, et de préférence 3 à 8 parties en poids de TiO$_2$ rutile pour 100 parties de PVC non additivé.

Les compositions stabilisantes selon l'invention, outre le produit brut réactionnel et/ou les résidus lourds de recristallisation et l'acétylacétonate, peuvent également contenir une quantité efficace d'au moins un additif notamment choisi parmi :

a) un sulfate et/ou un carbonate d'aluminium et/ou de magnésium notamment du type hydrotalcite. De tels produits sont par exemple décrits dans les brevets US-A-4 299 759, US-A-4 427 816, EP-A-453 379 et EP-A-509 864 ;

b) un aluminosilicate de métal alcalin, cristallin, synthétique, présentant une teneur en eau comprise entre 13 et 25 % en poids, de composition 0,7-1,1M$_2$O.Al$_2$O$_3$.1,3-2,4SiO$_2$ dans laquelle M symbolise un métal alcalin, notamment le sodium (DE-A-4 134 325 et US-A-4 540 233) ; comme en particulier les zéolites de type NaA, telles que décrites par exemple dans le brevet US-A-4 590 233 ;

c) un alcool ou un polyol organique conformément à l'enseignement de FR-A-2 356 674 et FR-A-2 356 695, et/ou un isocyanate (DE-A-4 134 325) ;

d) un sel d'un métal choisi parmi le calcium, le baryum, le magnésium et le strontium (EP-A-391 311) ;

e) un composé organique du zinc (EP-A-391 811) ;

f) un phosphite organique notamment des phosphites de trialkyle ou d'alkyle et de phényle ou de triphényle (EP-A-391 811) ou un complexe phosphite-hydroxy-aluminium-calcium tel que défini dans DE-A-4 134 325 ou encore un phosphite de calcium tel que décrit dans US-A-5 084 499 ;

g) des époxydes qui sont généralement des composés complexés, habituellement des polyglycérides époxydés, l'huile de lin époxydée, les huiles de poisson époxydées,

h) des adjuvants usuels tels que des antioxydants phénoliques, des agents anti-UV tels que les benzophénones, les benzotriazoles ou les amines stériquement encombrées (habituellement connues sous le terme de Hals).

Selon une variante particulièrement intéressante de l'invention, le produit brut (a) est broyé suivant une granulométrie similaire à celle de l'additif stabilisant supplémentaire le plus fin contenu dans la composition stabilisante. Cet additif peut être par exemple un carbonate et/ou un sulfate d'aluminium et/ou de magnésium, par exemple du type hydrotalcite et présenter alors une granulométrie inférieure à 100 μm comprise généralement entre 1 et 50 μm ou bien être un stéarate de calcium et/ou de baryum et présenter alors également une granulométrie inférieure à 100 μm, comprise généralement entre 10 et 100 μm.

De manière générale tout type de PVC convient, quel que soit son mode de préparation : polymérisation en masse, en suspension, en émulsion ou de tout autre type et quelle que soit sa visosité intrinsèque.

Les homopolymères du chlorure de vinyle peuvent également être modifiés chimiquement, par exemple par chloration. De nombreux copolymères du chlorure de vinyle peuvent également être stabilisés contre les effets de la chaleur, c'est-à-dire le jaunissement et la dégradation. Ce sont en particulier les copolymères obtenus par copolymérisation du chlorure de vinyle avec d'autres monomères présentant une liaison éthylénique polymérisable, comme par exemple l'acétate de vinyle ou le chlorure de vinylidène ; les acides maléique ou fumarique ou leurs esters ; les oléfines telles que l'éthylène, le propylène, l'hexène ; les esters acryliques ou méthacryliques ; le styrène ; les éthers vinyliques tels que le vinyldodécyléther.

Habituellement ces copolymères contiennent au moins 50 % en poids de motifs chlorure de vinyle et de préférence au moins 80 % en poids de motifs chlorure de vinyle.

Les compositions selon l'invention peuvent également contenir des mélanges à base de polymère chloré contenant des quantités minoritaires d'autres polymères, comme les polyoléfines halogénées ou les copolymères acrylonitrile-butadiène-styrène.

Le PVC seul ou en mélange avec d'autres polymères est le polymère chloré le plus largement utilisé selon l'invention.

Les compositions de l'invention sont plus particulièrement appropriées à la préparation de formulations rigides, c'est-à-dire sans plastifiant, ou semi-rigides, c'est-à-dire avec des teneurs en plastifiant réduites, telles que pour les applications dans le bâtiment, la fabrication de profilés divers, la fabrication de bouteilles.

Ces formulations contiennent le plus souvent un renforçateur de chocs, tel que les copolymères laurylacrylate/méthylméthacrylate, ou butylacrylate/méthylméthacrylate.

Elles peuvent également être des formulations plastifiées telles que pour la fabrication de films à usage agricole.

Les plastifiants utilisés sont des composés connus tels que par exemple des phtalates d'alkyle. Le plus souvent utilisé est le phtalate de di(éthyl-2-hexyle) (habituellement appelé phtalate de dioctyle).

Lorsque les compositions contiennent un plastifiant, la teneur de celui-ci est généralement de 5 % à 120 % en poids par rapport au poids de polymère chloré.

Toutes les méthodes usuelles d'incorporation des différents stabilisants ou adjuvants dans le polymère peuvent être utilisées. Par exemple l'homogénéisation de la composition polymérique peut être réalisée sur malaxeur ou mélangeur à rouleaux, à une température telle que la composition devienne fluide, normalement entre 150°C et 200°C pour le PVC et pendant une durée suffisante, de l'ordre de quelques minutes à quelques dizaines de minutes.

De manière habituelle, le taux d'incorporation des différents stabilisants ou adjuvants est faible par rapport au polymère chloré.

Il peut être ainsi avantageux de préparer un mélange préalable de 2 ou plusieurs des composés constitutifs des compositions selon l'invention avant leur incorporation dans le polymère chloré.

Les compositions de polymère chloré, et plus particulièrement de PVC, peuvent être mises en oeuvre selon toutes les techniques utilisées habituellement comme par exemple l'extrusion, l'injection, l'extrusion-soufflage, le calandrage ou le moulage par rotation.

Les exemples qui suivent illustrent l'invention.

Dans ce qui suit, ou ce qui précède sauf indications expresses contraires, les parties et pourcentages sont en poids.

**EXEMPLES 1 : Préparation du produit brut réactionnel P1.**

Dans un réacteur de 2000 cm$^3$ muni d'un réfrigérant, d'une bonne agitation et avec la possibilité d'être relié soit au vide soit à une source d'azote on introduit 260 ml de toluène puis sous couverture d'azote, 78 g de NaNH$_2$.

La température du milieu est ensuite portée à 40°C, puis maintenue durant toute la réaction et la finition à cette température.

L'ensemble de l'appareillage est mis sous une pression de 7 10$^4$ Pa.

On coule 310 g de stéarate de méthyle technique (contenant 10 % de palmitate de méthyle).

En 3 heures, on coule 120 g d'acétophénone.

La coulée de l'acétophénone étant terminée, on laisse sous agitation pendant 45 mn le milieu réactionnel (température de 40°C et sous pression de 7 10$^4$ Pa).

La solution toluénique est ensuite coulée à chaud dans une solution d'acide sulfurique dilué à 10 % de façon que le pH de la couche aqueuse après décantation soit de 1,5.

Après deux lavages, la solution toluénique est ensuite évaporée par hydrodistillation à pression atmosphérique. Puis le résidu est passé sur un tambour tournant refroidi en permanence pour conduire à un produit brut P1 sous forme "d'écailles", soit 420 g d'un produit solide à 20°C titrant 78 % en béta-dicétones (rendement 82 %, analyse chromatographique CPG).

**EXEMPLE 2 : Préparation d'une poudre P1.**

Dans un broyeur à marteau équipé d'une grille de 1 mm, on introduit au moyen d'un transporteur à vis 1 partie/heure de produit P1 de l'exemple 1 et 0,3 partie/heure d'azote liquide. Le produit broyé est récupéré par un transport pneumatique vers un système de séparation à filtre et l'on obtient une poudre blanche de granulométrie de 50 µm.

On obtient une poudre analogue en remplaçant l'azote liquide par du CO$_2$ liquide.

**EXEMPLE 3 : Stabilité thermique.**

On part de la composition en PVC (formulation rigide) suivante :

- PVC poudre préparé par polymérisation en suspension et commercialisé sous la dénomination S110P (ATOCHEM) : 100 parties
- Stéarate de calcium, stabilisant commercialisé par ATOCHEM sous la référence STAVINOR PSME : 0,3 "
- Stéréate de Zinc, stabilisant commercialisé par ATOCHEM sous la référence ZN70. : 1 "
- Didécylphénylphosphite, stabilisant commercialisé par CIBA-GEIGY sous la référence IRGASTAB CH 300 : 0,5 "
- Alcool polyvinylique, stabilisant commercialisé par RP CHIMIE sous la référence Rhodiastab PVAL : 0,2 "
- Hydrotalcite (hydrogénocarbônate d'aluminium et de magnésium), stabilisant commercialisé sous la référence ALCAMIZER 4 par MITSUI : 0,6 "
- Carbonate de Calcium broyé, charge commercialisée sous la référence OMYALITE 95T par OMYA : 5 "
- Pigment d'oxyde de Titane rutile de granulométrie comprise entre 0,1 et 0,5 µm : 6 "

- Renforçant choc (polymère acrylique), commercialisé par RHOM et HAAS sous le nom PARALOID KM 355 :      6,5 "
- Lubrifiants commercialisés par HENKEL sous les noms :

   .   LOXIOL G60 (ester d'un diacide aromatique et d'un alcool gras aliphatique) :      0,4 "
   .   LOXIOL G22 (cire de parafine) :      0,2 "

- d'un processing aid (agent de mise en oeuvre) commercialisé par ROHM et HAAS PARALOID :      1 "

A partir de cette composition, on réalise 3 échantillons en ajoutant :

$E_1$/0,30 partie du produit P1 de l'exemple 2
$E_2$/0,30 partie du produit P2 (P2 = acétylacétonate de calcium)
$E_3$/0,1 partie du produit P1 et 0,2 partie du produit P2 (formulation conforme à l'invention)
Chaque échantillon est homogénéisé dans un mélangeur rapide type Papenmeier à la vitesse de 1 800 tr/min jusqu'à une température de 115°C.

A partir de ces poudres, on procède à une évaluation de la stabilité thermique dynamique au plastographe BRABENDER ®. Cet appareillage est constitué :

- d'un système moteur électrique accouplé à un variateur continu ou discontinu de vitesse (0 à 200 t/min) ;
- d'un bain thermostaté à régulation de température proportionnelle électronique par de l'huile silicone ;
- d'un malaxeur, muni d'une cuve à double paroi, permettant un chauffage par circulation de l'huile silicone et de 2 rotors fixés par un système de verrouillage à baïonnette ;
- d'un chronomètre.

Chaque essai est introduit dans ce malaxeur à 150°C, à raison de 53 g, à l'aide d'une trémie et d'un piston poussé par un poids de 5 kg. Puis, toutes les 5 minutes, on effectue un prélèvement afin d'obtenir une pastille, jusqu'au noircissement ou cramage du mélange PVC.

Sur chaque pastille, on mesure par coloration à l'aide d'un chromamètre MINOLTA CR 200 ®, l'indice de jaunissement, paramètre b du système (L, a, b).

On obtient ainsi la dégradation thermique de chaque formule $E_1$, $E_2$ ou $E_3$ en fonction du temps.

Les résultats obtenus sont rassemblés dans le tableau 1 ci-après :

TABLEAU 1

| Temps | $E_1$ | $E_2$ | $E_3$ |
|---|---|---|---|
| 5 min | 3,3 | 2,9 | 2,9 |
| 10 min | 4,6 | 4,1 | 3,9 |
| 15 min | 5,1 | 5,2 | 5,1 |
| 20 min | 5,8 | 15,8 | 12,9 |

L'examen du tableau 1 montre que l'échantillon $E_1$ a une stabilité thermique meilleure que l'échantillon $E_2$.

La composition $E_3$ présente à la fois la bonne coloration initiale (à 5 minutes) de la formule $E_2$ et la bonne stabilité de coloration de la formule $E_1$.

## EXEMPLE 4 : Couleur et brillance.

On prépare 3 échantillons $E_1$, $E_2$ et $E_3$ comme à l'exemple 3.

A partir de ces poudres, on procède à une transformation par extrusion afin d'obtenir des plaques.

Les caractéristiques de l'extrudeuse monovis sont :

- Fabricant : ANDOUART,
- Vis conique :

   taux de compression = 2,8

rapport longueur/diamètre = 20
diamètre D = 40 mm

Les conditions d'extrusion sont :

- Vitesse de rotation de la vis = 23 tr/mn
- Profil de température :

| Zone 1 | 2 | 3 | Filières |
|--------|-----|-----|----------|
| 175°C | 180°C | 185°C | 190°C |

On mesure les propriétés colorimétriques suivant le système colorimétrique (L, a, b) CIE décrit précédemment à l'exemple 3 et la brillance à un angle d'incidence d'une valeur de 60°.

Les résultats obtenus sont rassemblés dans le tableau 2 suivant :

### TABLEAU 2

|  | $E_1$ | $E_2$ | $E_3$ |
|---|-------|-------|-------|
| L | 94,1 | 94,9 | 94,8 |
| a | 1,53 | 0,76 | 0,70 |
| b | 8,78 | 4,50 | 4,60 |
| Brillance | 45 | 45 | 57 |

On déduit de ce tableau 2 qu'en ce qui concerne la couleur, $E_2$ et $E_3$ sont similaires et meilleurs que $E_1$. Par contre, au niveau de la brillance, $E_3$ est bien meilleur que $E_1$ et $E_2$.

### EXEMPLE 5 : Rosissement.

On procède comme à l'exemple 4.

Les plaques extrudées sont soumises à un test de rosissement dans les conditions suivantes :

Les échantillons sont soumis à un cycle de rayonnement UV de 200 heures dans les conditions UVCON :

- Appareillage UVCON de ATLAS
- spectre d'éclairement : UVA avec un maximum à $\lambda$ = 340 nm et filtre < 290 nm
- Température de corps noir = 55°C

Puis, ils sont laissés 24 heures sans rayonnement UV dans une étuve ventilée à 70°C.

On obtient ainsi la sensibilité au rosissement de chaque formule en fonction du temps. Cette sensibilité est mesurée par la variation $\Delta a$, a étant le paramètre précité du système (L, a, b) CIE.

Les résultats obtenus sont rassemblés dans le tableau 3 ci-dessous :

|  | $E_1$ | $E_2$ | $E_3$ |
|---|---|---|---|
| $\Delta a$ | 3,00 | 0,70 | 0,90 |

Il ressort de ce tableau 3 que les formulations $E_2$ et $E_3$ ont une faible sensibilité au phénomène de rosissement contrairement à $E_1$.

Au vu des différentes valeurs d'usage exemplifiées ci-dessus, il apparaît que la composition $E_3$ conforme à l'invention offre le meilleur compromis entre la stabilité thermique, la couleur et le rosissement (tenue à la lumière).

**Revendications**

1. Procédé pour stabiliser une composition pour polymère chloré, **caractérisé en ce qu'**il comprend le fait d'incorporer à titre de composition stabilisante :

    a) le produit brut susceptible d'être obtenu en mettant en oeuvre les étapes suivantes :

    - réaction de condensation d'un ester sur une cétone en présence d'un agent alcalin,
    - acidification du mélange réactionnel,
    - au moins un lavage à l'eau du mélange ainsi obtenu, et
    - élimination du solvant, le produit brut comportant entre 10 et 95 % en poids de béta-dicétone,

    b) un complexe métallique d'acétylacétonate,

    le rapport des constituants a/b étant compris entre 50/50 et 1/99.

2. Procédé selon la revendication 1, **caractérisé en ce que** le produit brut issu de l'étape a) comprend 20 à 80 % en poids de béta-dicétone.

3. Procédé pour stabiliser une composition pour polymère chloré, **caractérisé en ce qu'**il comprend le fait d'incorporer à titre de composition stabilisante :

    a) le produit brut susceptible d'être obtenu en mettant en oeuvre les étapes suivantes :

    - réaction de condensation d'un ester sur une cétone en présence d'un agent alcalin,
    - acidification du mélange réactionnel,
    - au moins un lavage à l'eau du mélange ainsi obtenu
    - élimination du solvant,
    - recristallisation du produit brut comportant entre 10 et 95% en poids de β-dicétone dans un solvant organique approprié,
    - séparation du produit recristallisé essentiellement formé de béta-dicétones des jus-mères par filtration, et
    - évaporation du solvant des jus-mères pour obtenir un résidu solide de recristallisation, et

    b) mélange dudit résidu solide de recristallisation avec un complexe métallique d'acétylacétonate,

    le rapport des constituants a/b étant compris entre 50/50 et 1/99.

**4.** Procédé selon la revendication 3, **caractérisé en ce que** le résidu solide de recristallisation comprend entre 20 et 40 % de béta-dicétones.

**5.** Procédé selon l'une quelconque des revendications 1 à 4, **caractérisé en ce que** la réaction de condensation est la suivante :

$$R_1COCHR_2H + R_3CO\text{-}OR_4 + ACat \text{ ----> } R_1COCHR_2COR_3 + R_4OH$$

dans laquelle :

- ACat est choisi parmi un amidure d'un cation ou un hydrure d'un cation ;
- $R_1$ et $R_3$ sont identiques ou différents et représentent chacun un radical hydrocarboné, ayant de 1 à 30 atomes de carbone ;
- $R_2$ est un hydrogène ou un radical hydrocarboné, présentant au plus 4 atomes de carbone ;
- $R_1$ et $R_2$ peuvent être reliés de manière à former un cycle ;
- $R_4$ représente un radical hydrocarboné.

**6.** Procédé selon la revendication 5, **caractérisé en ce que** $R_1$ et $R_3$ sont identiques ou différents et représentent chacun un radical hydrocarboné ayant de 1 à 18 atomes de carbone.

**7.** Procédé selon la revendication 5, **caractérisé en ce que** $R_2$ est un radical alkyle présentant au plus 4 atomes de carbone.

**8.** Procédé selon l'une quelconque des revendications 5 à 7, **caractérisé en ce que** $R_1$ et $R_3$, qui sont identiques ou différents, représentent :

- un radical aralkyle ou alcényle, linéaire ou ramifié, ayant jusqu'à 24 atomes de carbone ;
- un radical aralkyle ayant de 7 à 10 atomes de carbone ;
- un radical aryle ou cycloaliphatique ayant moins de 14 atomes de carbone ;

et $R_4$ représente un radical alkyle ayant de 1 à 4 atomes de carbone.

**9.** Procédé selon la revendication 8, **caractérisé en ce que** $R_4$ représente un radical méthyle.

**10.** Procédé selon l'une quelconque des revendications précédentes, **caractérisé en ce que** l'ester est le stéarate de méthyle, l'acétone est l'acétophénone et l'agent alcalin est l'amidure de sodium.

**11.** Procédé selon l'une quelconque des revendications précédentes, **caractérisé en ce que** le constituant (a) est sous forme solide.

**12.** Procédé selon la revendication 11, **caractérisé en ce que** le constituant (a) est sous forme de poudre de granulométrie inférieure à 500 μm.

**13.** Procédé selon la revendication 12, **caractérisé en ce que** le constituant a) est sous forme de poudre de granulométrie inférieure à 200 μm.

**14.** Procédé selon la revendication 12 ou 13, **caractérisé en ce que** la poudre a) est obtenue à partir du produit brut solide à 20°C et non purifié, par la mise en oeuvre d'au moins l'une des techniques suivantes :

- précipitation dans un solvant,
- broyage cryogénique,
- pulvérisation / séchage dans un courant froid à co-courant ou à contre-courant.

**15.** Procédé selon l'une quelconque des revendications précédentes, **caractérisé en ce que** le constituant b) est sous forme d'un complexe de calcium ou de zinc.

**16.** Procédé selon l'une quelconque des revendications précédentes, **caractérisé en ce que** le rapport des constituants a/b est compris entre 20/80 et 1/99.

**17.** Procédé selon l'une quelconque des revendications précédentes, **caractérisé en ce qu'**on introduit en outre de 0,5 à 10 parties en poids d'oxyde de titane rutile.

**18.** Procédé selon la revendication 17, **caractérisé en ce qu'**on introduit de 3 à 8 parties en poids d'oxyde de titane rutile.

**19.** Procédé selon la revendication 17 ou 18, **caractérisé en ce que** l'oxyde de titane rutile présente une granulométrie comprise entre 0,1 et 0,5 µm.

**20.** Procédé selon l'une quelconque des revendications précédentes, **caractérisé en ce que** l'on introduit en outre un additif stabilisant supplémentaire choisi parmi un carbonate et/ou sulfate d'aluminium et/ou de magnésium, un aluminosilicate de métal alcalin, du stéarate de calcium et/ou de baryum.

**21.** Procédé selon la revendication 20, **caractérisé en ce que** l'additif stabilisant est une hydrotalcite.

**22.** Procédé pour l'obtention d'objets conformes à partir d'une composition pour polymère chloré, **caractérisé par** la mise en oeuvre du procédé tel que défini selon l'une quelconque des revendications 1 à 21.

**23.** Procédé selon l'une des revendications 1 à 21 **caractérisé en ce que** l'élimination du solvant est réalisé par évaporation.

**24.** Procédé selon l'une des revendications 1 à 21 **caractérisé en ce que** l'élimination du solvant est réalisé par écaillage.

**25.** Procédé selon la revendication 24, **caractérisé en ce que** l'écaillage est réalisé par passage du mélange réactionnel sur un tambour tournant refroidi en permanence.

**Patentansprüche**

**1.** Verfahren zur Stabilisierung einer Zusammensetzung für ein chloriertes Polymer, **dadurch gekennzeichnet, dass** es den Einbau

a) eines Rohprodukts, das erhalten werden kann, indem folgende Stufen durchgeführt werden:

- Kondensieren eines Esters an ein Keton in Gegenwart eines alkalischen Mittels,
- Ansäuern des Reaktionsgemischs,
- mindestens einmaliges Waschen des so erhaltenen Gemischs mit Wasser und
- Entfernen des Lösungsmittels, wobei das Rohprodukt zwischen 10 und 95 Gew.% β-Diketon enthält, und

b) eines Acetylacetonatmetallkomplexes, wobei das Verhältnis der Bestandteile a/b 50/50 bis 1/99 beträgt,

als stabilisierende Zusammensetzung umfasst.

**2.** Verfahren nach Anspruch 1, **dadurch gekennzeichnet, dass** das in Stufe a) erhaltene Rohprodukt 20 bis 80 Gew.% β-Diketon enthält.

**3.** Verfahren zur Stabilisierung einer Zusammensetzung für ein chloriertes Polymer, **dadurch gekennzeichnet, dass** es den Einbau

a) eines Rohprodukts, das erhalten werden kann, indem folgende Stufen durchgeführt werden:

- Kondensieren eines Esters an ein Keton in Gegenwart eines alkalischen Mittels,
- Ansäuern des Reaktionsgemischs,
- mindestens einmaliges Waschen des so erhaltenen Gemischs mit Wasser,
- Entfernen des Lösungsmittels,

- Umkristallisieren des Rohprodukts, das zwischen 10 und 95 Gew.% β-Diketon enthält, aus einem geeigneten organischen Lösungsmittel,
- Abtrennen des im Wesentlichen aus β-Diketonen gebildeten umkristallierten Produkts von den Mutterlaugen durch Filtration und
- Verdampfen des Lösungsmittels der Mutterlaugen, um einen festen Umkristallisationsrückstand zu erhalten, und

b) eines Gemischs dieses festen Umkristallisationsrückstands mit einem Acetylacetonatmetallkomplex, wobei das Verhältnis der Bestandteile a/b 50/50 bis 1/99 beträgt,

als stabilisierende Zusammensetzung umfasst.

4. Verfahren nach Anspruch 3, **dadurch gekennzeichnet, dass** der feste Umkristallisationsrückstand 20 bis 40 Gew. % β-Diketone enthält.

5. Verfahren nach einem der Ansprüche 1 bis 4, **dadurch gekennzeichnet, dass** die Kondensation folgende ist:

$$R_1COCHR_2H + R_3CO\text{-}OR_4 + ACat \rightarrow R_1COCHR_2COR_3 + R_4OH,$$

in welcher

- ACat aus einem Amid oder Hydrid eines Kations ausgewählt ist,
- $R_1$ und $R_2$ gegebenenfalls voneinander verschieden sind und jeweils einen Kohlenwasserstoffrest mit 1 bis 30 Kohlenstoffatomen bedeuten,
- $R_2$ einen Wasserstoff- oder Kohlenwasserstoffrest, der höchstens 4 Kohlenstoffatome besitzt, bedeutet,
- $R_1$ und $R_2$ derart verbunden sein können, dass sie einen Cyclus bilden, und
- $R_4$ einen Kohlenwasserstoffrest bedeutet.

6. Verfahren nach Anspruch 5, **dadurch gekennzeichnet, dass** $R_1$ und $R_3$ gegebenenfalls voneinander verschieden sind und jeweils einen Kohlenwasserstoffrest mit 1 bis 18 Kohlenstoffatomen bedeuten.

7. Verfahren nach Anspruch 5, **dadurch gekennzeichnet, dass** $R_2$ einen Alkylrest bedeutet, der höchstens 4 Kohlenstoffatome besitzt.

8. Verfahren nach einem der Ansprüche 5 bis 7, **dadurch gekennzeichnet, dass** $R_1$ und $R_3$, die gegebenenfalls voneinander verschieden sind,

- einen geradkettigen oder verzweigten Aralkyl- bzw. Alkenylrest mit bis zu 24 Kohlenstoffatomen,
- einen Aralkylrest mit 7 bis 10 Kohlenstoffatomen und
- einen cycloaliphatischen oder Arylrest mit weniger als 14 Kohlenstoffatomen bedeuten und
- $R_4$ einen Alkylrest mit 1 bis 4 Kohlenstoffatomen bedeutet.

9. Verfahren nach Anspruch 8, **dadurch gekennzeichnet, dass** $R_4$ den Methylrest bedeutet.

10. Verfahren nach einem der vorhergehenden Ansprüche, **dadurch gekennzeichnet, dass** der Ester Methylstearat, das Aceton Acetophenon und das alkalische Mittel Natriumamid ist.

11. Verfahren nach einem der vorhergehenden Ansprüche, **dadurch gekennzeichnet, dass** der Bestandteil a) in fester Form vorliegt.

12. Verfahren nach Anspruch 11, **dadurch gekennzeichnet, dass** der Bestandteil a) in Form eines Pulvers mit einer Korngröße von unter 500 μm vorliegt:

13. Verfahren nach Anspruch 12, **dadurch gekennzeichnet, dass** der Bestandteil a) in Form eines Pulvers mit einer Korngröße von unter 200 μm vorliegt.

14. Verfahren nach Anspruch 12 oder 13, **dadurch gekennzeichnet, dass** das Pulver a) aus dem bei 20°C festen Rohprodukt, das nicht gereinigt worden ist, durch mindestens eines der folgenden Verfahren:

- Ausfällen aus einem Lösungsmittel,
- Zermahlen bei tiefen Temperaturen und
- Pulverisieren/Trocknen in einem kalten Gleich- oder Gegenstrom

erhalten ist.

15. Verfahren nach einem der vorhergehenden Ansprüche, **dadurch gekennzeichnet, dass** der Bestandteil b) in Form eines Calcium- oder Zinkkomplexes vorliegt.

16. Verfahren nach einem der vorhergehenden Ansprüche, **dadurch gekennzeichnet, dass** das Verhältnis der Bestandteile a/b 20/80 bis 1/99 beträgt.

17. Verfahren nach einem der vorhergehenden Ansprüche, **dadurch gekennzeichnet, dass** außerdem 0,5 bis 10 Gew.-Tl. Titanoxid (Rutil) zugegeben werden.

18. Verfahren nach Anspruch 17, **dadurch gekennzeichnet, dass** 3 bis 8 Gew.-Tl. Titanoxid (Rutil) zugegeben werden,

19. Verfahren nach Anspruch 17 oder 18, **dadurch gekennzeichnet, dass** das Titanoxid (Rutil) eine Korngröße von 0,1 bis 0,5 $\mu$m besitzt.

20. Verfahren nach einem der vorhergehenden Ansprüche, **dadurch gekennzeichnet, dass** außerdem ein zusätzliches Stabilisierungsadditiv zugegeben wird, das aus einem Carbonat und/oder Sulfat des Aluminiums und/oder Magnesiums, einem Alkalimetallaluminosilicat, Calcium- und/oder Bariumstearat ausgewählt wird.

21. Verfahren nach Anspruch 20, **dadurch gekennzeichnet, dass** das Stabilisierungsadditiv Hydrotalkit ist.

22. Verfahren zur Herstellung von Gegenständen, die aus einer Zusammensetzung für ein chloriertes Polymer geformt werden, **dadurch gekennzeichnet, dass** das Verfahren wie in einem der Ansprüche 1 bis 21 definiert angewendet wird.

23. Verfahren nach einem der vorhergehenden Ansprüche 1 bis 21, **dadurch gekennzeichnet, dass** die Entfernung des Lösungsmittels durch Verdampfen erfolgt.

24. Verfahren nach einem der Ansprüche 1 bis 21, **dadurch gekennzeichnet, dass** die Entfernung des Lösungsmittels durch Erstarrenlassen erfolgt.

25. Verfahren nach Anspruch 24, **dadurch gekennzeichnet, dass** das Erstarrenlassen mittels Laufenlassen des Reaktionsgemischs über eine sich drehende Trommel erfolgt, die ständig gekühlt wird.

**Claims**

1. Process for stabilising a composition for chlorinated polymer, **characterised in that** the following are incorporated as a stabilising composition:

a) the crude product which can be obtained by means of the following stages:

- condensation reaction of an ester on a ketone in the presence of an alkaline agent,
- acidification of the reaction mixture,
- washing the mixture thus obtained at least once in water, and
- eliminating the solvent, the crude product having between 10 and 95% by weight of beta-diketone;

b) a metallic acetylacetonate complex,

the ratio of the constituents a:b being between 50:50 and 1:99 inclusive.

2. Process according to Claim 1, **characterised in that** the crude product from stage a) comprises 20 to 80% by weight of beta-diketone.

3. Process for stabilising a composition for chlorinated polymer, **characterised in that** the following are incorporated as a stabilising composition:

   a) the crude product which can be obtained by means of the following stages:

   - condensation reaction of an ester with a ketone in the presence of an alkaline agent,
   - acidification of the reaction mixture,
   - washing the mixture thus obtained at least once in water,
   - eliminating the solvent,
   - recrystallisation of the crude product which has between 10 and 95% by weight of beta-diketone in a suitable organic solvent,
   - separating the recrystallised product formed from beta-diketones from the mother liquors by filtration, and
   - evaporating the solvent from the mother liquors to obtain a solid recrystallisation residue, and

   b) mixing the said solid recrystallisation residue with a metallic acetylacetonate complex,

   the ratio of the constituents a:b being between 50:50 and 1:99 inclusive

4. Process according to Claim 3, **characterised in that** the solid recrystallisation residue includes between 20 and 40% beta-diketones.

5. Process according to Claims 1 to 4, **characterised in that** the condensation reaction is as follows:

$$R_1COCHR_2H + R_3CO\text{-}OR_4 + ACat \rightarrow R_1COCHR_2COR_3 + R_4OH$$

in which:

   - ACat is chosen from among an amide of a cation or a hydride of a cation;
   - $R_1$ and $R_3$ are identical or different and each represents a hydrocarbon radical, having from 1 to 30 carbon atoms;
   - $R_2$ is a hydrogen or a hydrocarbon radical, having up to 4 carbon atoms;
   - $R_1$ and $R_2$ can be linked so as to form a ring;
   - $R_4$ represents a hydrocarbon radical.

6. Process according to Claim 5, **characterised in that** $R_1$ and $R_3$ are identical or different and each represents a hydrocarbon radical with 1-18 carbon atoms.

7. Process according to Claim 5, **characterised in that** $R_2$ is an alkyl radical having up to 4 carbon atoms.

8. Process according to any one of the Claims 5 to 7, **characterised in that** $R_1$ and $R_3$, which are identical or different, represent:

   - an aralkyl or alkenyl radical, linear or branched, with up to 24 carbon atoms;
   - an aralkyl radical with 7 to 10 carbon atoms;
   - an aryl or cycloaliphatic radical with fewer than 14 carbon atoms;
   - and $R_4$ represents an alhyl radical with 1 to 4 carbon atoms.

9. Process according to Claim 8, **characterised in that** $R_4$ represents a methyl radical.

10. Process according to any one of the foregoing claims, **characterised in that** the ester is methyl stearate, the acetone is acetophenone and the alkaline agent is sodium amide.

11. Process according to any one of the foregoing claims, **characterised in that** the constituent (a) is in solid form.

12. Process according to Clam 11, **characterised in that** the constituent (a) is in the form of powder with a particle size smaller than 500μm.

13. Process according to Claim 12, **characterised in that** the constituent (a) is in the form of powder with a particle size smaller than 200μm.

14. Process according to Claim 12 or 13, **characterised in that** the powder (a) is obtained from the solid crude product at 20°C, unpurified, using at least one of the following techniques:

    - precipitation in a solvent,
    - cryogenic crushing
    - spray-drying in a cold stream with combined flow or counterflow.

15. Process according to any one of the foregoing claims, **characterised in that** constituent (b) is in the form of a calcium or zinc complex.

16. Process according to any one of the foregoing claims, **characterised in that** the ratio of the constituents a:b is between 20:80 and 1:99.

17. Process according to any one of the foregoing claims, **characterised in that** 0.5 to 10 parts by weight of rutile titanium oxide is also introduced.

18. Process according to Claim 17, **characterised in that** 3 to 8 parts by weight of rutile titanium oxide is introduced.

19. Process according to Claim 17 or 18, **characterised in that** the rutile titanium oxide has a particle size between 0.1 and 0.5 μm inclusive.

20. Process according to any one of the foregoing claims, **characterised in that** an extra stabilising additive is also introduced, chosen from among an aluminium and/or magnesium carbonate and/or sulphate, aluminosilicate of alkaline metal, calcium and/or barium stearate.

21. Process according to Claim 20, **characterised in that** the stabilising additive is a hydrotalcite.

22. Process for obtaining conforming items from a composition for chlorinated polymer, **characterised by** using the process as defined according to any one of the Claims 1 to 21.

23. Process according to one of the Claims 1 to 21, **characterised in that** the solvent is eliminated by evaporation.

24. Process according to one of the Claims 1 to 21, **characterised in that** the solvent is eliminated by flaking.

25. Process according to Claim 24, **characterised in that** the flaking is done by passing the reaction mixture over a continuously cooled rotating drum.